# EUROPEAN PATENT SPECIFICATION

(11) **EP 2 329 834 B1**
(45) Date of publication and mention of the grant of the patent: **12.06.2019**
(21) Application number: 09810061.3
(22) Date of filing: 28.08.2009
(51) Int. Cl.: A23K 10/18, A23K 50/10, A23K 50/75, A23K 50/40, A23L 33/135

(54) **ANTICOCCIDIUM COMPOSITION**
ANTIKOKZIDIEN-ZUSAMMENSETZUNG
COMPOSITION ANTI-COCCIDIE

(30) Priority: 29.08.2008 JP 2008220906
(43) Date of publication of application: 08.06.2011
(73) Proprietor: Meiji Feed Co., Ltd., Chiyoda-ku Tokyo 101-0041 (JP); Meiji Co., Ltd., Chuo-ku Tokyo 104-8306 (JP)
(72) Inventor: SERA, Kenji, Saitama-shi Saitama 330-0061 (JP); MASHIKO, Takanori, Mito-shi Ibaraki 310-0801 (JP); IKEGAMI, Shuji, Odawara-shi Kanagawa 250-0862 (JP); ORIHASHI, Takenori, Mito-shi Ibaraki 310-0836 (JP); KANBE, Michio, Higashimurayama-shi Tokyo 189-0003 (JP)
(74) Representative: Fédit-Loriot
(86) International application number: PCT/JP2009/065120
(87) International publication number: WO 2010/024413

(56) References cited:
- EP-A1- 1 611 796
- EP-A1- 1 867 238
- EP-A1- 2 194 118
- GB-A- 2 170 984
- JP-A- 2004 305 128
- JP-A- 2006 254 841
- US-A- 2 465 905
- NIPPON BISEIBUTSUGAKU KYOKAI BISEIBUTSUGAKU JITEN 23 August 1989, page 293, XP008144676

## Description

### Technical Field

The present disclosure relates to a system for preventing and/or treating coccidiosis in humans and/or animals excluding humans, and more particularly relates to an anticoccidial agent, a feed for controlling a coccidiosis containing the same, a method for controlling coccidiosis comprising orally administering the same to humans and/or animals excluding humans, and a method for rearing animals excluding humans while controlling coccidiosis.

### Background Art

Coccidiosis is a gastrointestinal protozoan infection in which diarrhea is the primary symptom. This is a disease developed by the parasitism of protozoa called "coccidium" having a size of 0.01 mm to 0.05 mm in the intestinal tract (small intestine to large intestine) of humans and animals such as cattle, chickens, dogs, and cats along with various factors . Most of the coccidiosis is a complex infection and symptoms do not appear only by infection with coccidia, however, a factor such as stress acts as a trigger and diarrhea or the like appears.

In young animals, diarrhea is one of the diseases causing most serious economic loss in livestock farmers. For example, in the case of cattle, it is said that the yearly economic loss in the US due to diarrhea in calves is from 10 to 50 billion yen or more, and also in Japan, diarrhea in calves incurs extremely serious damage to the economy.

Animals infected with a protozoa causing coccidiosis such as a protozoan of the genus Eimeria suffer from symptoms such as diarrhea or bloody stools, and if an appropriate treatment is not given or if the symptoms are severe, the animals sometimes die. Even if the animals do not die, the growth of the animals is significantly inhibited. Further, coccidiosis is one of the diseases causing damage particularly to animals in the neonatal and juvenile periods, and if the growth is stagnated due to the occurrence of diarrhea, the productivity thereafter was adversely affected, and therefore, it becomes a big problem.

Further, it has been reported that the detection rate of coccidia is as much as 35% in a group of Japanese black calves in which diarrhea frequently occurs. On the other hand, also in the case of piglets, it has been reported that the detection rates of coccidia are 21.6% in a group of suckling piglets suffering from diarrhea and 14.7% in a group of weaning piglets suffering from diarrhea. Therefore, the coccidium is a major cause of the occurrence of diarrhea in young animals regardless of animal species.

A coccidial protozoa proliferates in the mucosa of the gastrointestinal tract and excretes an infectious body covered with a hard shell called "oocyst" in the feces. The coccidial protozoa spreads to another animal by oral infection with this excreted oocyst.

As measures for coccidiosis, it is generally recommended that a livestock barn is disinfected with an ortho drug (for example, a crehazol agent) or by scalding (steaming) or flaming. However, the oocyst of coccidium has extremely high resistance to heat and chemicals, and therefore, it is difficult to remove the oocyst from the environment by using a synthetic fungicide or under a heat sterilization condition to be used for common measures for microorganisms.

In the prevention or treatment of coccidiosis, an antibiotic (a polyether antibiotic such as salinomycin, or the like), a chemotherapeutic agent composed of a synthetic antimicrobial agent (a sulfa drug, or the like) and a biological drug such as a vaccine have been mainly used. However, such an antibiotic or a chemotherapeutic agent has problems such as the occurrence of side effects and a decline in the effect due to acquisition of resistance to the agent, and a vaccine is used only for the prevention and cannot be used for the treatment. Moreover, in the case where humans take the meat, milk, of the animal to which such an agent has been administered, there is a problem of migration in the human body of the agent remaining in the animal body, and a strict limitation on the using amount and administration period thereof has been required.

Accordingly, there has been a strong demand for an agent which is derived from a natural substance and therefore is highly safe and has an excellent preventive and therapeutic effect on coccidiosis without problems such as those of the above-mentioned antibiotic, synthetic antimicrobial agent, vaccine, etc.

As for chickens, the following methods for preventing or treating coccidiosis using the so-called probiotics have been known: a method in which chickens are experimentally infected with Eimeria acervulina which is one of the causes of coccidiosis, and then fed a feed containing live lactic acid bacteria, thereby increasing the diseased cell-mediated immunity so as to reduce the excretion of Eimeria acervulina oocysts from the feces (Non-patent document 1); a method for treating coccidiosis in poultry including chickens by orally administering powdered viable cells of lactic acid bacteria (Pediococcus acidilactici) or the like to modulate the in vivo immune response (Patent document 1).

However, as for a lactic acid bacterium belonging to Lactobacillus gasseri, the anticoccidial effect by a probiotics has not been known, and also there have not been reported the anticoccidial effect (the anticoccidial effect of the so-called prebiotics in which indigenous lactic acid bacteria in the animal intestinal tract are proliferated) of a fermentation product thereof.

Further, as for a propionic acid bacterium belonging to Propionibacterium freudenreichii, the anticoccidial effect of a probiotics has not at all been known. Also, the anticoccidial effect (the anticoccidial effect of the so-called prebiotics) of a fermentation product thereof (particularly, a fermentation product obtained by using a medium containing whey) has not at all been reported.

When examining the prior art, as a material having the so-called prebiotic effect, the followings are known: an oligosaccharide, a fermentation product of a lactic acid bacterium, and a fermentation product of a propionic acid bacterium. It is known that the fermentation product of a lactic acid bacterium has a prebiotic effect such as an effect of improving the intestinal microflora of livestock, an effect of preventing diarrhea, or an effect of increasing the body weight (Patent document 2) . It is also known that the fermentation product of a propionic acid bacterium has an effect of improving the intestinal microflora by promoting the proliferation of an intestinal bifidobacterium (Patent document 3). However, a prebiotic material which is reported to have a preventive or therapeutic effect on coccidiosis has not been found at the present.

Further, the preventive and/or therapeutic effect (i.e., the anticoccidial effect and controlling effect on coccidiosis) by use of both substances together on coccidiosis have not at all been known in the past, and the present invention is the first to disclose the effect.

The "probiotics" as used herein refers to viable cells capable of improving the balance of the intestinal flora formed by intestinal microorganisms or a food containing the same. The "prebiotics" as used herein refers to an indigestible component capable of promoting the proliferation of useful intestinal bacteria or controlling the proliferation of harmful bacteria, or a food containing the same. Incidentally, an indigestible component which is produced by microbial fermentation and shows a bioactivity in vivo directly or through intestinal microorganisms, or a food containing the same is sometimes referred to as a "biogenics".

### Prior Art Documents

### Patent Documents

Patent document 1: JP-A-2008-044945
Patent document 2: JP-A-2004-305128
Patent document 3: JP-A-2006-254841

Further, GB 2 170 984 A and US 2 465 905 A disclose compositions comprising a fermentation product of both lactic acid bacteria and propionic acid bacteria, respectively.

### Non-Patent Document

Non-patent document 1: Avian diseases, 47(4), 1313-1320 (2003)

### Summary of the Invention

### Problems that the Invention is to Solve

The present invention has been made for the purpose of providing an orally administrable agent for medical use for humans and/or animals excluding humans (particularly, livestock, poultry, etc.) for use in the treatment of coccidiosis, which is derived from a natural substance, can be orally administered over a long period of time, and is highly safe with no side effects, an agent for a food or a drink for use in the preservation of health, a feed additive, and a feed comprising the same.

### Means for Solving the Problems

To the above end, there is provided a mixture for use in the treatment of coccidiosis, characterized by comprising, as active ingredients,
(A) at least one fermentation product selected from the group consisting of lactic acid bacterial cells obtainable by neutralization culturing Lactobacillus gasseri OLL 2716 strain (FERM BP-6999) in a whey protein derivative-containing medium, a culture of said lactic acid bacterium, a broken cell of said lactic acid bacterium, a residue of a culture of said lactic acid bacterium and a product obtainable by treating said lactic acid bacterial cells, said culture, said broken cells and said residue of a culture; and
(B) at least one fermentation product selected from the group consisting of propionic acid bacterial cells obtainable by culturing Propionibacterium freudenreichii ET-3 strain (FERM BP-8115) in a medium containing whey and/or protease-treated whey, a culture of said propionic acid bacterium, a broken cell of said propionic acid bacterium, a residue of a culture of said propionic acid bacterium, and a product obtainable by treating said propionic acid bacterial cells, said culture, said broken cells and said residue of a culture.

Preferably, the product obtained by treating the lactic acid bacterial cells, the culture, the broken cells and the residue of a culture is at least one product selected from the group consisting of a concentrated product, a pasted product, a dried product, a liquid product, a diluted product, and a sterilized product.

Preferably still, the product obtained by treating the propionic acid bacterial cells, the culture, the broken cells and the residue of a culture is at least one product selected from the group consisting of a concentrated product, a pasted product, a dried product, a liquid product, a diluted product, and a sterilized product.

Preferably yet, the fermentation product of a lactic acid bacterium or of a propionic acid bacterium comprises fermentation liquids obtainable by treating the culture of the lactic acid bacterium or of the culture of the propionic acid bacterium by solid-liquid separation and/or dried products thereof, respectively.

Preferably further, the whey protein derivative-containing medium contains at least one of a whey protein concentrate i.e. WPC, a whey protein isolate i.e. WPI, and a hydrolysate of them.

Preferably, the mixture is administered to calves against the coccidiosis caused by protozoa of the genus Eimeria.

Preferably still, the mixture is administered over a period longer than 25 days after birth.

Preferably yet, the mixture is administered over a period between 30 and 90 days after birth.

### Advantage of the Invention

According to the present invention, by orally administering a fermentation product of a lactic acid bacterium and/or a whey fermentation product of a propionic acid bacterium, these products act as an orally administrable preventive or therapeutic agent for medical use for humans, livestock, poultry, rodents, small animals for pets, animals housed in zoos (so-called companion animals), or an agent for a food or a drink for use in the preservation of health, and coccidiosis (including a mixed infection) can be prevented or diarrhea caused by coccidiosis can be treated. In particular, for livestock or poultry, etc., by rearing such animals by administering these fermentation products directly or through feeding a feed in which the fermentation products are blended (preferably for a period longer than the neonatal period, for example for 25 days after birth to 60 days after birth), it was confirmed that the occurrence of coccidiosis can be prevented.

The anticoccidial agent according to the present invention was dissolved in substitute milk, and the resulting substitute milk was administered to calves immediately after birth for 60 days. As a result, the occurrence of coccidiosis was observed in the group of control calves to which the anticoccidial agent was not administered, but the occurrence of coccidiosis was not observed in the group of test calves although the test calves were reared with the control calves, and it was confirmed that the occurrence of highly infectious coccidiosis is inhibited.

### Brief Description of the Drawings

Fig. 1 is a graph showing the total number of days per calf suffering from diarrhea (bloody stools) during the test period.
Fig. 2 is a graph showing the total number of days per calf suffering from diarrhea (bloody stools) treated by an antiparasitic agent or rehydration during the test period.

### Best Mode for Carrying Out the Invention

The fermentation product of a lactic acid bacterium according to the present invention is at least one of a culture of a lactic acid bacterium (the whole culture including cells, a medium, metabolic secretions) obtained by culturing a lactic acid bacterium belonging to Lactobacillus gasseri (for example, Lactobacillus gasseri OLL 2716 strain) , cells (viable cells or dead cells) isolated therefrom, a broken cell (a broken cell of the lactic acid bacterium), a residue (including a culture liquid or a culture supernatant which is not a turbid portion of the culture liquid but a clear portion thereof) of the culture of the lactic acid bacterium obtained by removing solid matters including the cells from the culture, and a treated product thereof. The "treated product thereof" as used herein refers to at least one selected from a concentrated product, a pasted product, a dried product (at least one of a spray-dried product, a freeze-dried product, a vacuum-dried product, and a drum-dried product), a liquid product, a diluted product, and a sterilized product.

Incidentally, the above-mentioned Lactobacillus gasseri OLL 2716 strain was internationally deposited on January 14, 2000 as deposition No. FERM BP-6999 in the National Institute of Bioscience and Human-Technology, Agency of Industrial Science and Technology, Ministry of International, Trade and Industry, at present, the International Patent Organism Depositary, National Institute of Advanced Industrial Science and Technology (Zip code: 305-8566, Central 6, 1-1-1 Higashi, Tsukuba, Ibaraki, Japan).

Lactobacillus gasseri may be cultured according to a common procedure suitable for culturing of a lactic acid bacterium or bacterial species, however, as the medium, a synthetic medium or a semi-synthetic medium which facilitates the isolation of cells by centrifugal separation is preferably used. As the culturing method, neutralization culturing is preferably carried out while keeping the pH within a defined range (for example, pH 4.0 to 8.0) until the number of cells reaches a predetermined value. In general, the culturing is performed at 20 to 40°C (preferably at 35 to 38°C) for about 24 to 48 hours. By the high-concentration culturing method, a culture rich in cells and metabolites can be obtained.

As the medium, a synthetic medium, a semi-synthetic medium, or another commonly used medium (for example, LBS broth (manufactured by Japan Becton, Dickinson and Company)) can be suitably used. As a preferred high-concentration culture solution for Lactobacillus gasseri, a medium that is added a whey protein derivative and/or lactose, particularly a medium composed mainly of a whey protein derivative and/or lactose (a protein content of 0.1 to 10% and/or a lactose content of 1 to 15%) to above described medium is used, and neutralization culturing is performed at 30 to 45°C while keeping the pH at 4.5 to 7.0 until 10⁸ to 10¹¹ cells, preferably 10⁹ to 10¹⁰ cells are obtained. Incidentally, in the case of using a conventional culturing method, the number of the cells in the culture reaches only 10⁹ or less.

As the whey protein derivative, at least one selected from a whey protein concentrate (WPC), a whey protein isolate (WPI), and a hydrolysate thereof is used, and a commercially available product can also be suitably used.

WPC is obtained by treating whey, which is generated in the production of cheese or casein, by ultrafiltration, gel filtration, lactose crystal separation, so as to increase the protein content to 35 to 85% (in terms of solid content) . WPI is different from WPC and has a protein content of about 95% (in terms of solid content) which is increased by an ion exchange method.

As the hydrolysate, an enzyme-treated product obtained by treating at least one selected from WPC, WPI, whey, and a whey protein with a protease such as pepsin, trypsin, or papain is used.

The fermentation product of a lactic acid bacterium includes a product composed only of Lactobacillus gasseri cells and other substances derived from Lactobacillus gasseri, and also a product obtained by adding an excipient (starch, dextrin, a milk component, silicic acid), water, a feed component, thereto. This fermentation product can be formed into any of a liquid, a paste, and a solid. This fermentation product can be administered to young animals by feeding the product as such, and also can be administered by adding the product to a feed or drinking water and feeding young animals with the resulting feed or drinking water.

In the case of administering the fermentation product of a lactic acid bacterium to, for example, calves, the daily dose thereof per calf is from 0.93 g to 92.52 g (0.1 to 10 g in terms of solid content), preferably from 4.65 g to 46.3 g (0.5 g to 5 g in terms of solid content) . In the case of other animals, the dose may be increased or decreased in proportion to body weight.

As the residue of a culture of a lactic acid bacterium, for example, the whole culture (containing about 15 billion cells/mL) obtained by culturing L. gasseri OLL 2716 strain (FERM BP-6999) in a WPC-containing medium as described above is centrifuged, and the resulting liquid portion (supernatant portion) is sterilized, and the sterilized liquid can be used. Further, a dried product obtained by drying the sterilized liquid can also be used.

On the other hand, the whey fermentation product of a propionic acid bacterium which is the other active ingredient according to the present invention is at least one of a culture of a propionic acid bacterium (the whole culture including cells, a medium, metabolic secretions) obtained by culturing, for example, a bacterium belonging to Propionibacterium freudenreichii which produces a bifidogenic growth stimulator (BGS) in a medium containing whey (whey and/or protease-treated whey), cells (living cells or dead cells) isolated therefrom, a homogenate of the cells (a broken cell of the propionic acid bacterium), a residue (including a culture liquid or a culture supernatant which is not a turbid portion of the culture liquid but a clear portion thereof) of the culture of the propionic acid bacterium obtained by removing solid matters including the cells from the culture, and a treated product thereof. The "treated product thereof" as used herein refers to at least one selected from a concentrated product, a pasted product, a dried product (at least one of a spray-dried product, a freeze-dried product, a vacuum-dried product, and a drum-dried product), a liquid product, a diluted product, and a sterilized product.

Examples thereof include a fermentation liquid (culture supernatant) obtained by fermenting a 10% whey powder reduction liquid with Propionibacterium freudenreichii ET-3 strain and a fermentation product of a propionic acid bacterium (Profec: ILSI, No. 80, 5-13 (2004)).

Incidentally, the above-mentioned Propionibacterium freudenreichii ET-3 strain was internationally deposited on July 11, 2002 as deposition No. FERM BP-8115 in the International Patent Organism Depositary, National Institute of Advanced Industrial Science and Technology (Zip code: 305-8566, Central 6, 1-1-1 Higashi, Tsukuba, Ibaraki, Japan) .

The whey fermentation product of a propionic acid bacterium (Propionibacterium freudenreichii ET-3 Culture, Profec) was approved to indicate that the product can proliferate bifidobacteria to improve intestinal environment and to keep bowel movement favorable as a food for specified health use (TOKUHO) for milk drinks and tablets in 2001 and 2002.

However, the anticoccidial effect of the Profec has not at all been known, and there have been no findings that the anticoccidial effect is confirmed by actually administering the Profec to young animals such as calves. Also, there have been no known reported cases of the reduction in the number of treatments by controlling the occurrence of serious diarrhea accompanied by bleeding using the Profec, and the present invention is the first to disclose the effect. Further, the excellent anticoccidial effect of a combination use thereof with the fermentation product of a lactic acid bacterium have not at all been known in the past, and this new finding was found for the first time by the present invention. Accordingly, it can be said that the present invention succeeded in developing the so-called second medicinal effect (second usage) newly.

As the propionic acid bacterium, a bacterium which produces BGS is suitably used, however, the bacterium is not limited to this. Examples thereof include Propionibacterium freudenreichii ET-3 strain(above-mentioned), Propionibacterium freudenreichii ATCC 6207 strain, Propionibacterium freudenreichii ATCC 8262 strain, Propionibacterium freudenreichii IFO 12424 strain, Propionibacterium freudenreichii IFO 12426 strain, and Propionibacterium freudenreichii IFO 12391 strain.

The propionic acid bacterium may be cultured aerobically or anaerobically (for example, under pressure with nitrogen gas (0.5 kg/cm²)) according to a common procedure except for using a medium containing whey (whey and/or protease-treated whey having a protein content of about 1%). The whey content in the medium is preferably from 1 to 20%, more preferably from 5 to 15%. In general, liquid culturing may be performed at 20 to 40°C (preferably at 35 to 38°C) at a pH of about 6.0 to 8.0 for about 24 to 48 hours.

The whey fermentation product of a propionic acid bacterium includes a product composed only of Propionibacterium freudenreichii cells and other substances derived from Propionibacterium freudenreichii, and also a product obtained by adding an excipient (starch, dextrin, a milk component, silicic acid, water, a feed component, or the like thereto. This fermentation product can be formulated into any of a liquid, a paste, and a solid. The fermentation product can be administered to young animals by feeding the product as such, and also can be administered by adding the product to a feed or milk replacer and feeding young animals with the resulting feed or milk replacer.

As described below, the dose of the whey fermentation product of a propionic acid bacterium is extremely small and does not impose a burden on target animals such as young animals. The present invention is extremely unique in terms of this point.

In the case of administering the whey fermentation product of a propionic acid bacterium, for example, the Profec to calves, the daily dose thereof per calf is from 0.04 g to 10 g, preferably from 0.1 g to 4 g. In this manner, this fermentation product exhibits an excellent effect sufficiently in an extremely small amount, and if desired, this fermentation product may be given not in a single dose but in divided doses bid, tid, etc. Incidentally, in the case of using a whey fermentation product of a propionic acid bacterium other than the Profec, or in the case of administering the product to other animals, a necessary dose may be suitably determined based on the above description.

In the present invention, the fermentation product of a lactic acid bacterium and the whey fermentation product of a propionic acid bacterium may be used in combination. These fermentation products may be used separately, or a mixture of these fermentation products (a material containing both fermentation products) may be used. The mixing ratio of the fermentation product of a lactic acid bacterium to the whey fermentation product of a propionic acid bacterium is preferably from 1:10 to 100:1, more preferably from 1:5 to 50:1 (dry weight ratio).

As the mixture of these fermentation products, a commercially available product can also be used. As the commercially available product, for example, Prebio Support (registered trademark of Meiji Feed Co., Ltd., PS), which is a powder product obtained by formulating a mixture of a residue of a culture of Lactobacillus gasseri OLL 2716 strain (FERM BP-6999) (LG) and the Profec into a preparation using dextrin, lactose, and silica can be used. In the PS, the fermentation product of a lactic acid bacterium and the whey fermentation product of a propionic acid bacterium are contained at a ratio (dry weight ratio) of 8.5:1.5 to 9.5:0.5. That is, the mixing ratio of the fermentation product of a lactic acid bacterium to the whey fermentation product of a propionic acid bacterium is such that the fermentation product of a lactic acid bacterium is contained in an amount of 85 to 95% (% by dry weight) of the total amount of both fermentation products, and the rest is the whey fermentation product of a propionic acid bacterium.

It is considered that the fermentation product of a lactic acid bacterium yields cell components and metabolites (organic acids, peptides, oligosaccharides, bacteriocin, oligo-DNA, etc.) and the physiological effect such as antibacterial effect and immunostimulatory effect of these components organically work to exhibit an anticoccidial effect. The present invention succeeded in exhibiting a desired excellent anticoccidial effect by using the fermentation product of a lactic acid bacterium, the effect of which is not sufficient alone, in combination with the fermentation product of a propionic acid bacterium.

The anticoccidial composition according to the present invention contains the fermentation product of a lactic acid bacterium and the fermentation product of a propionic acid bacterium as active ingredients and can be used as a food or a drink, a feed, a pharmaceutical, or a veterinary pharmaceutical.

In the case where the composition is used as a food or a drink, the composition can be suitably used in a conventional manner such that the present active ingredients are used as such or the composition is used in combination with another food or food component. The agent according to the present invention using the present active ingredients may be in any form of a solid (a powder, a granule, and others), a paste, a liquid, and a suspension, however, it is preferred that the present active ingredients are formulated into an anticoccidial drink (for preventing coccidiosis) using a sweetener, a sour agent, a vitamin, and any of various components commonly used for the production of a drinkable preparation.

The same shall apply to the case where the composition is used as a feed, and the composition can be suitably used in a conventional manner such that the present active ingredients are used as such or the composition is used in combination with another feed or feed component. Further, it is also possible to use the composition by adding the present active ingredients to drinking water or milk replacer.

In the case where the composition is used as a pharmaceutical or a veterinary pharmaceutical, though it can be parenterally administered, the composition is generally orally administered. Examples of the preparation for oral administration include a tablet, a pill, a granule, a soft or hard capsule, a powder, a fine granule, an emulsion, a suspension, a syrup, and an elixir. These various preparations can be formulated in a conventional manner by adding a known formulation aid which can commonly used in the pharmaceutical formulation technical field such as an excipient, a binder, a disintegrant, a lubricant, a corrigent, a solubilizing agent, a suspension agent, or a coating agent to the principal agent.

As described above, the present invention was finally completed as a result of intensive studies of the effectiveness of the fermentation product of a lactic acid bacterium and the whey fermentation product of a propionic acid bacterium. According to the present invention, coccidiosis in animals can be prevented or diarrhea caused by coccidiosis can be treated. In particular, for livestock or poultry, etc., by rearing such animals by administering these fermentation products directly or through feeding a feed in which the fermentation products are blended, the occurrence of coccidiosis can be prevented. As the rearing method, it is desirable to perform feeding thereof for preferably a period longer than the neonatal period (preferably for a period longer than the neonatal period by 60 days or more, for example, for a period longer than the neonatal period by 80 to 100 days).

Incidentally, in the above description, a case of cattle which are livestock is mainly described as an example, however, the present invention can also be applied to livestock such as chickens, pigs, sheep, and horses besides cattle; poultry; and rodents such as mice, rats, guinea pigs, hamsters, and ferrets; and also can be applied to small animals for pets such as dogs and cats; animals housed in zoos (so-called companion animals); and humans.

Hereinafter, the present invention will be specifically described with reference to Examples, however, the technical scope of the present invention is not limited to these Examples.

### Examples

37 newborn calves (Japanese Black Cattle) were given artificial IgG-enriched colostrum immediately after birth until 3 days old. The calves were divided into 2 groups when they were 4 days old, and the calves in a test group (18 calves) were given milk replacer containing Prebio Support (registered trademark of Meiji Feed Co., Ltd., PS) such that the dose of Prebio Support was 20 g/day, and the calves in a control group (19 calves) were given only milk replacer until the calves reached 60 days old. Thereafter, only the calves in the test group were given a feed (50 g/day) obtained by formulating the PS into tablets until the calves reached 90 days old. During the test period, all of the calves were given a concentrate for calves (starter) ad libitum, and the health conditions of the calves and the number of times of treatments were recorded.

Incidentally, Prebio Support (PS) is a trade name of Meiji Feed Co., Ltd. and is a powder product obtained by formulating a mixture of a residue of a culture of Lactobacillus gasseri OLL 2716 strain (FERM BP-6999) (LG) and a fermentation product of Propionibacterium freudenreichii ET-3 strain (FERM BP-8115) (a whey fermentation product of a propionic acid bacterium) into a preparation using dextrin, lactose, and silicon dioxide. In this Example, a product in which the mixing ratio of the LG to the whey fermentation product of a propionic acid bacterium is 9:1 was used.

Incidentally, as the artificial colostrum, a product of Bayer Medical Co., Ltd. (trade name: Head Start (registered trademark), component value: protein (45%)) having an IgG content of 26.7% (60 g/225 g) was used. As the milk replacer , a product of Meiji Feed Co., Ltd. (trade name: Premium Meirac (registered trademark of Meiji Dairies Corporation), component values: protein (24%), fat (20%)) containing milk protein and vegetable oil and fat as main ingredients was used. As the mixed feed for calves, a product of Meiji Feed Co., Ltd. (trade name: Premium Meistarter, component values: protein (20%), total digestable nutrients (75%)) containing corn and soybean meal as main ingredients was used.

As a result, during the test period, 3 calves among the 19 calves in the control group developed bloody stools (incidence rate: 15.8%), however, the 18 calves in the test group developed no bloody stools (incidence rate: 0.0%) (Table 1). At this time, each of the calves which developed bloody stools was examined and it was confirmed that bloody stools was caused by coccidiosis in all the cases.

**[Table 1]**

| | Number of calves developing diarrhea (number of calves developing diarrhea/number of calves in group) | Incidence rate (%) |
|---|---|---|
| Control group | 3/19 | 15.8 |
| Test group | 0/18 | 0.0 |

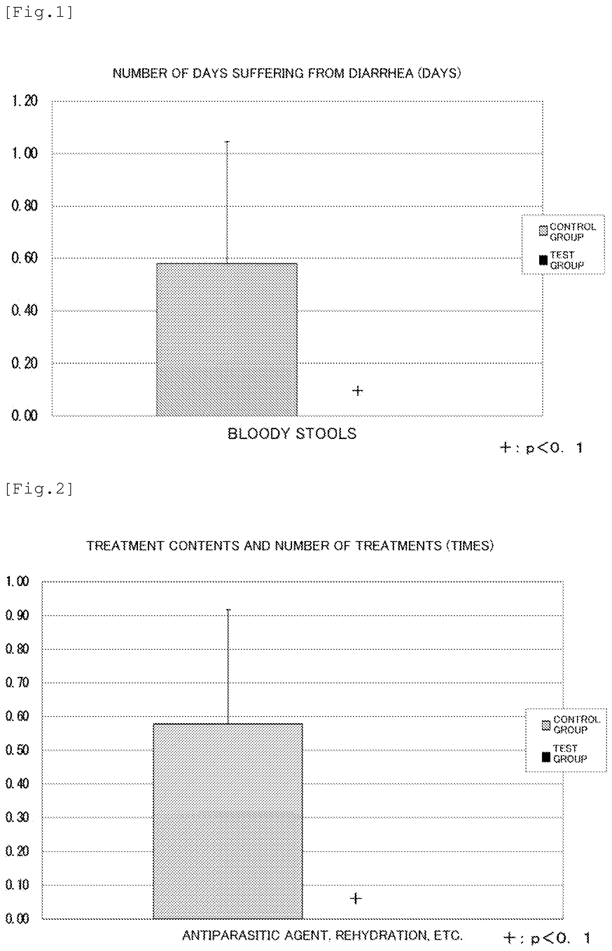

Further, the average number of days per calf suffering from bloody stools during the test period was 0.58 in the control group, however, it was 0 in the test group (Fig. 1 or Table 2). Further, the average total number of treatments per calf required for coccidial diarrhea such as an antiparasitic agent or rehydration during the test period was 0.58 in the control group, however, it was 0 in the test group (Fig. 2 or Table 2).

**[Table 2]**

| | Average number of days suffering from diarrhea (days) | Average number of treatments (times) |
|---|---|---|
| Control group | 0.58 | 0.58 |
| Test group | 0 | 0 |
| P-value | 0.086 | 0.086 |

Accordingly, it was found that by administering a material as disclosed in present claims (mixture) containing both of the fermentation product of a lactic acid bacterium and the whey fermentation product of a propionic acid bacterium through feeding, the occurrence of coccidiosis in a normal rearing environment was controlled, and it was revealed that these active ingredients have an anticoccidial effect.

## Claims

1. A mixture for use in the treatment of coccidiosis, **characterized by** comprising, as active ingredients,
(A) at least one fermentation product selected from the group consisting of lactic acid bacterial cells obtainable by neutralization culturing Lactobacillus gasseri OLL 2716 strain (FERM BP-6999) in a whey protein derivative-containing medium, a culture of said lactic acid bacterium, a broken cell of said lactic acid bacterium, a residue of a culture of said lactic acid bacterium and a product obtainable by treating said lactic acid bacterial cells, said culture, said broken cells and said residue of a culture; and
(B) at least one fermentation product selected from the group consisting of propionic acid bacterial cells obtainable by culturing Propionibacterium freudenreichii ET-3 strain (FERM BP-8115) in a medium containing whey and/or protease-treated whey, a culture of said propionic acid bacterium, a broken cell of said propionic acid bacterium, a residue of a culture of said propionic acid bacterium, and a product obtainable by treating said propionic acid bacterial cells, said culture, said broken cells and said residue of a culture.

2. The mixture for use according to claim 1, wherein the at least one fermentation product of (A) includes a product composed only of the cells of Lactobacillus gasseri OLL 2716 strain (FERM BP-6999) and other substances derived from Lactobacillus gasseri OLL 2716 strain (FERM BP-6999).

3. The mixture for use according to claim 1 or 2, wherein the at least one fermentation product of (A) includes a product obtained by adding starch, dextrin, a milk component, silicic acid, water or a feed component thereto.

4. The mixture for use according to any one of claims 1 to 3, wherein the at least one fermentation product of (B) includes a product composed only of the cells of Propionibacterium freudenreichii ET-3 strain (FERM BP-8115) and other substances derived from Propionibacterium freudenreichii ET-3 strain (FERM BP-8115).

5. The mixture for use according to any one of claims 1 to 4, wherein the at least one fermentation product of (B) includes a product obtained by adding starch, dextrin, a milk component, silicic acid, water or a feed component thereto.

6. The mixture for use according to any one of claims 1 to 5, wherein said product obtained by treating said lactic acid bacterial cells, said culture, said broken cells and said residue of a culture is at least one product selected from the group consisting of a concentrated product, a pasted product, a dried product, a liquid product, a diluted product, and a sterilized product.

7. The mixture for use according to any one of claims 1 to 6, wherein said product obtained by treating said propionic acid bacterial cells, said culture, said broken cells and said residue of a culture is at least one product selected from the group consisting of a concentrated product, a pasted product, a dried product, a liquid product, a diluted product, and a sterilized product.

8. The mixture for use according to any one of claims 1 to 7, wherein said fermentation product of a lactic acid bacterium or of a propionic acid bacterium comprises fermentation liquids obtainable by treating the culture of the lactic acid bacterium or of the culture of the propionic acid bacterium by solid-liquid separation and/or dried products thereof, respectively.

9. The mixture for use according to any one of claims 1 to 8, wherein said whey protein derivative-containing medium contains at least one of a whey protein concentrate i.e. WPC, a whey protein isolate i.e. WPI, and a hydrolysate of them.

10. The mixture for use according to any one of claims 1 to 9, **characterized in that** the mixture is administered to calves against the coccidiosis caused by protozoa of the genus Eimeria.

11. The mixture for use according to claim 10, **characterized in that** the mixture is administered over a period longer than 25 days after birth.

12. The mixture for use according to claim 10, **characterized in that** the mixture is administered over a period between 30 and 90 days after birth.

## Patentansprüche

1. Gemisch zur Verwendung bei der Behandlung von Kokzidiose, **gekennzeichnet durch** einen Gehalt an folgenden Wirkstoffen:
(A) mindestens ein Fermentationsprodukt, ausgewählt aus der Gruppe, die besteht aus Milchsäurebakterien-Zellen, erhältlich durch Neutralisationszüchtung von Lactobacillus gasseri OLL 2716-Stamm (FERM BP-6999) in einem ein Molkeprotein-Derivat enthaltenden Medium, einer Kultur dieses Milchsäurebakteriums, einer aufgebrochenen Zelle dieses Milchsäurebakteriums, einem Rückstand einer Kultur dieses Milchsäurebakteriums und einem Produkt, das durch Behandlung dieser Milchsäurebakterien-Zellen, dieser Kultur, dieser aufgebrochenen Zellen und dieses Rückstands einer Kultur erhalten worden ist; und
(B) mindestens ein Fermentationsprodukt, ausgewählt aus der Gruppe, die besteht aus Propionsäurebakterien-Zellen, erhältlich durch Züchtung von Propionibacterium freudenreichii ET-3-Stamm (FERM BP-8115) in einem Medium, das Molke und/oder mit Protease behandelte Molke enthält, einer Kultur dieses Propionsäurebakteriums, einer aufgebrochenen Zelle dieses Propionsäurebakteriums, einem Rückstand einer Kultur dieses Propionsäurebakteriums und einem Produkt, das durch Behandlung dieser Propionsäurebakterien-Zellen, dieser Kultur, dieser aufgebrochenen Zellen und dieses Rückstands einer Kultur erhalten worden ist;

2. Gemisch zur Verwendung nach Anspruch 1, wobei das mindestens eine Fermentationsprodukt von (A) ein Produkt umfasst, das nur aus den Zellen von Lactobacillus gasseri OLL 2716-Stamm (FERM BP-6999) und anderen Substanzen, die von Lactobacillus gasseri OLL 2716-Stamm (FERM BP-6999) abgeleitet sind, zusammengesetzt ist.

3. Gemisch zur Verwendung nach Anspruch 1 oder 2, wobei das mindestens eine Fermentationsprodukt von (A) ein Produkt umfasst, das durch Zugabe von Stärke, Dextrin, einer Milchkomponente, Kieselsäure, Wasser oder einer Futtermittelkomponente erhalten worden ist.

4. Gemisch zur Verwendung nach einem der Ansprüche 1 bis 3, wobei das mindestens eine Fermentationsprodukt von (B) ein Produkt umfasst, das nur aus den Zellen von Propionibacterium freudenreichii ET-3-Stamm (FERM BP-8115) und anderen Substanzen, die von Propionibacterium freudenreichii ET-3-Stamm (FERM BP-8115) abgeleitet sind, zusammengesetzt ist.

5. Gemisch zur Verwendung nach einem der Ansprüche 1 bis 4, wobei das mindestens eine Fermentationsprodukt von (B) ein Produkt umfasst, das durch Zugabe von Stärke, Dextrin, einer Milchkomponente, Kieselsäure, Wasser oder einer Futtermittelkomponente erhalten worden ist.

6. Gemisch zur Verwendung nach einem der Ansprüche 1 bis 5, wobei es sich bei dem Produkt, das durch Behandlung dieser Milchsäurebakterien-Zellen, dieser Kultur, dieser aufgebrochenen Zellen und dieses Rückstands einer Kultur erhalten worden ist, um mindestens ein Produkt handelt, das aus der Gruppe ausgewählt ist, die besteht aus einem konzentrierten Produkt, einem pastenartigen Produkt, einem getrockneten Produkt, einem flüssigen Produkt, einem verdünnten Produkt und einem sterilisierten Produkt.

7. Gemisch zur Verwendung nach einem der Ansprüche 1 bis 6, wobei es sich bei dem Produkt das durch Behandlung dieser Propionsäurebakterien-Zellen, dieser Kultur, dieser aufgebrochenen Zellen und dieses Rückstands einer Kultur erhalten worden ist, um mindestens ein Produkt handelt, das aus der Gruppe ausgewählt ist, die besteht aus einem konzentrierten Produkt, einem pastenartigen Produkt, einem getrockneten Produkt, einem flüssigen Produkt, einem verdünnten Produkt und einem sterilisierten Produkt.

8. Gemisch zur Verwendung nach einem der Ansprüche 1 bis 7, wobei das Fermentationsprodukt eines Milchsäurebakteriums oder eines Propionsäurebakteriums Fermentationsflüssigkeiten, die durch Behandlung einer Kultur des Milchsäurebakteriums oder des Propionsäurebakteriums durch Fest-Flüssig-Trennung erhalten worden sind, und/oder getrocknete Produkte davon umfasst.

9. Gemisch zur Verwendung nach einem der Ansprüche 1 bis 8, wobei das ein Molkeprotein-Derivat enthaltende Medium mindestens einen der folgenden Bestandteile enthält: ein Molkeprotein-Konzentrat, d. h. WPC, ein Molkeprotein-Isolat, d. h. WPI, und ein Hydrolysat davon.

10. Gemisch zur Verwendung nach einem der Ansprüche 1 bis 9, **dadurch gekennzeichnet, dass** das Gemisch Kälbern gegen die durch Protozoen der Gattung Eimeria verursachte Kokzidiose verabreicht wird.

11. Gemisch zur Verwendung nach Anspruch 10, **dadurch gekennzeichnet, dass** das Gemisch über einen Zeitraum von mehr als 25 Tagen nach der Geburt verabreicht wird.

12. Gemisch zur Verwendung nach Anspruch 10, **dadurch gekennzeichnet, dass** das Gemisch über einen Zeitraum von 30 bis 90 Tagen nach der Geburt verabreicht wird.

## Revendications

1. Mélange destiné à une utilisation pour le traitement de la coccidiose, **caractérisé par le fait qu'**il comprend, en tant qu'ingrédients actifs,
(A) au moins un produit de fermentation sélectionné à partir du groupe constitué par des cellules bactériennes d'acide lactique pouvant être obtenues par la neutralisation de culture de souche de lactobacillus Gasseri OLL 2716 (FERM BP-6999) dans un milieu contenant un dérivé de protéine de lactosérum, une culture de ladite bactérie d'acide lactique, un fragment de cellule de ladite bactérie d'acide lactique, un résidu d'une culture de ladite bactérie d'acide lactique et un produit pouvant être obtenu par traitement desdites cellules bactériennes d'acide lactique, de ladite culture, desdits fragments de cellule et dudit résidu de culture ; et
(B) au moins un produit de fermentation sélectionné à partir du groupe constitué par des cellules bactériennes d'acide propionique pouvant être obtenues en cultivant une souche de propionobactérie freudenreichii ET-3 (FERM BP-8115) dans un milieu contenant du lactosérum et/ou du lactosérum traité à la protéase, une culture de ladite bactérie d'acide propionique, un fragment de cellule de ladite bactérie d'acide propionique, un résidu d'une culture de ladite bactérie d'acide propionique, et un produit pouvant être obtenu par traitement desdites cellules bactériennes d'acide propionique, de ladite culture, desdits fragments de cellule et dudit résidu d'une culture.

2. Mélange destiné à une utilisation selon la revendication 1, dans lequel le au moins un produit de fermentation de (A) comporte un produit composé seulement des cellules de la souche de lactobacille Gasseri OLL 2716 (FERM BP-6999) et d'autres substances dérivées de la souche de lactobacille Gasseri OLL 2716 (FERM BP-6999).

3. Mélange destiné à une utilisation selon la revendication 1 ou 2, dans lequel le au moins un produit de fermentation de (A) comporte un produit obtenu en ajoutant de l'amidon, de la dextrine, un composant lacté, de l'acide silicique, de l'eau ou un composant alimentaire à ce dernier.

4. Mélange destiné à une utilisation selon l'une quelconque des revendications 1 à 3, dans lequel le au moins un produit de fermentation de (B) comporte un produit composé seulement des cellules de la souche de propionobactérie freudenreichii ET-3 (FERM BP-8115) et d'autres substances dérivées de la souche de propionobactérie freudenreichii ET-3 (FERM BP-8115).

5. Mélange destiné à une utilisation selon l'une quelconque des revendications 1 à 4, dans lequel le au moins un produit de fermentation de (B) comporte un produit obtenu en ajoutant de l'amidon, de la dextrine, un composant lacté, de l'acide silicique, de l'eau ou un composant alimentaire à ce dernier.

6. Mélange destiné à une utilisation selon l'une quelconque des revendications 1 à 5, dans lequel ledit produit obtenu par traitement desdites cellules bactériennes d'acide lactique, de ladite culture, desdits fragments de cellule et dudit résidu d'une culture est au moins un produit sélectionné à partir du groupe constitué par un produit concentré, un produit en pâte, un produit déshydraté, un produit liquide, un produit dilué et un produit stérilisé.

7. Mélange destiné à une utilisation selon l'une quelconque des revendications 1 à 6, dans lequel ledit produit obtenu par traitement desdites cellules bactériennes d'acide propionique, de ladite culture, desdits fragments de cellule et dudit résidu d'une culture est au moins un produit sélectionné à partir du groupe constitué par un produit concentré, un produit en pâte, un produit déshydraté, un produit liquide, un produit dilué, et un produit stérilisé.

8. Mélange destiné à une utilisation selon l'une quelconque des revendications 1 à 7, dans lequel ledit produit de fermentation d'une bactérie d'acide lactique ou d'une bactérie d'acide propionique comprend des liquides de fermentation pouvant être obtenus respectivement par traitement de la culture de la bactérie d'acide lactique ou de la culture de la bactérie d'acide propionique par séparation de solide-liquide et/ou des produits déshydratés de ceux-ci.

9. Mélange destiné à une utilisation selon l'une quelconque des revendications 1 à 8, dans lequel ledit milieu contenant un dérivé de protéine de lactosérum contient au moins l'un d'un concentré protéique de lactosérum c'est-à-dire du WPC, d'un isolat de protéine de lactosérum c'est-à-dire du WPI et d'un hydrolysât de ceux-ci.

10. Mélange destiné à une utilisation selon l'une quelconque des revendications 1 à 9, **caractérisé en ce que** le mélange est administré à des veaux contre la coccidiose provoquée par des protozoaires du genre Eimeria.

11. Mélange destiné à une utilisation selon la revendication 10, **caractérisé en ce que** le mélange est administré sur une période supérieure à 25 jours après la naissance.

12. Mélange destiné à une utilisation selon la revendication 10, **caractérisé en ce que** le mélange est administré sur une période comprise entre 30 et 90 jours après la naissance.
